# EUROPEAN PATENT SPECIFICATION

(11) **EP 3 600 558 B1**
(45) Date of publication and mention of the grant of the patent: **10.01.2024**
(21) Application number: 18718634.1
(22) Date of filing: 28.03.2018
(51) Int. Cl.: A61Q 1/00, A61Q 5/00, A61Q 13/00, A61Q 15/00, A61Q 19/00, A61K 8/85, A61K 8/92, A61K 8/02

(54) **COSMETIC COMPOSITION COMPRISING A BIODEGRADABLE POLYESTER AND AN OILY PHASE**
KOSMETISCHE ZUSAMMENSETZUNG MIT EINEM BIOLOGISCH ABBAUBAREN POLYESTER UND EINER ÖLPHASE
COMPOSITION COSMÉTIQUE COMPRENANT UN POLYESTER BIODÉGRADABLE ET UNE PHASE HUILEUSE

(30) Priority: 30.03.2017 IT 201700035338
(43) Date of publication of application: 05.02.2020
(73) Proprietor: BIO ON S.p.A., 10128 Torino (IT)
(72) Inventor: SAETTONE, Paolo, 40016 San Giorgio di Piano (BO) (IT); COMES FRANCHINI, Mauro, 40016 San Giorgio di Piano (BO) (IT)
(74) Representative: Bottero, Carlo
(86) International application number: PCT/IB2018/052135
(87) International publication number: WO 2018/178899

(56) References cited:
- EP-A1- 1 980 235
- CN-A- 105 418 944
- JP-A- 2003 073 233
- US-A1- 2015 231 042

## Description

The present invention relates to a cosmetic composition comprising a biodegradable polyester and an oily phase.

Polymers are a class of products widely used in cosmetics, in particular for the preparation of skin emulsions. For example, hydrophilic polymers are used in oil-in-water emulsions as rheological and thickening modifiers but also as stabilizers, emulsifiers and moisturizers.

Another function that can be performed by polymers of various nature in the form of fine powders is that of texturizing agents (also called "skin feel enhancers"), i.e. agents that improve the sensation of the consumer during the application of the cosmetic product to the skin, so as to make the cosmetic product more pleasant and acceptable, improving flowability and spreadability, reducing greasiness and giving the consumer a sensation of "rich" formulation, more suitable for the use for which it is intended. In other words, the addition of a texturizing agent, in order to increase the consistency of the cosmetic product, should at the same time ensure excellent flowability properties and a reduced greasiness.

The texturizing agent to be introduced into the formulation is first of all selected according to the type of cosmetic product in question (hydrogel, lipogel, emulsion, etc.) and can generally be a polymeric product, such as nylon, polymethylmethacrylates (PMMA), polyurethanes, silicones, or also an inorganic product, such as silica, talc, boron nitride, borosilicates.

For example, patent application US 2006/0204465 (on behalf of Seppic SA) relates to the use of copolymers or terpolymers of methylmethacrylate with different monomers (for example butyl acrylate and other acrylates) as texturizing agents in cosmetic or pharmaceutical compositions.

Patent application US 2010/0215701 (in the name of Arkema France) describes a cosmetic composition comprising a continuous water-based phase, a fatty phase and a phase in the form of porous powder. The latter consists of a copolyamide deriving from the condensation of various monomers (amino acids, lactams and others). Other patent applications, again in the name of Arkema France, describe cosmetic compositions where different polyamides are used as texturizing agents (see US 2013/0142747 and US 2013/0156833).

However, the presence of synthetic polymers in cosmetic products is considered an important factor in water pollution, so it is extremely important to replace these products with others that are biodegradable.

The Applicant has therefore posed the problem of developing formulations that contain biodegradable products as texturizing agents, which ensure the desired consistency and flowability properties to the cosmetic composition during the application, as well as a reduced greasiness.

The Applicant has now found that this problem and others that will be better illustrated hereafter can be solved by adding to the base cosmetic composition at least one polyhydroxyalkanoate (PHA) in the form of particles with an average diameter (d50) from 0.1 um to 100 um, preferably from 1 um to 50 um, in an amount of from 0.1% by weight to 30% by weight, preferably from 0.5% to 15% by weight, with respect to the total weight of the composition , wherein the particles of PHA are obtained by a process of atomization by spray drying of an aqueous suspension of PHA.

In a first aspect, the present invention therefore relates to a cosmetic composition which comprises:
from 70% by weight to 99.9% by weight of a cosmetically acceptable base formulation comprising an oily phase;
from 0.1% by weight to 30% by weight of at least one polyhydroxyalkanoate (PHA) in the form of particles having an average diameter (d50) from 0.1 µm to 100 µm;
the percentages being expressed with respect to the total weight of the composition , wherein the particles of PHA are obtained by a process of atomization by spray drying of an aqueous suspension of PHA.

Preferably, the PHA particles have a substantially spherical shape.

Preferably, the particles of PHA have an average diameter (d50) from 1 µm to 50 µm, more preferably from 5 µm to 30 µm.

The Applicant has found that such particles of PHA have a marked ability to absorb oily substances, whereby they make the cosmetic composition containing an oily phase particularly pleasant to the touch and during the application on the skin, minimizing the greasy effect. In addition, the PHA particles have the ability to absorb excess sebum, making the skin less shiny. The particles of PHA further contribute to the so-called "soft focus" effect, i.e. an immediate filling and smoothing effect on wrinkles and cutaneous furrows, giving a greater shine and compactness to the skin.

Furthermore, the Applicant has found that the inclusion of the particles of PHA in a mascara formulation clearly improves gloss, elongating and curling effect on the eyelashes.

Particularly advantageous effects have also been found in formulations for lipsticks, which are particularly compact and resistant to breakage, in addition to giving a particularly evident covering effect on the lips.

As is known, polyhydroxyalkanoates (PHA) are polymers produced by microorganisms isolated from natural environments or also from genetically modified microorganisms, and are characterized by a high biodegradability. They are produced and accumulated from various species of bacteria under unfavorable growth conditions and in the presence of an excess carbon source. PHAs are synthesized and accumulated from about 300 different microbial species, comprised in more than 90 types of Gram-positive and Gram-negative bacteria, such as *Bacillus, Rhodococcus, Rhodospirillum, Pseudomonas, Alcaligenes, Azotobacter, Rhizobium.*

In cells, PHA is stored in the form of microgranules, whose size and number per cell varies in different bacterial species. They appear under the electron microscope as refractive inclusions, with a diameter ranging from 0.2 to 0.7 um.

For the purposes of the present invention, the PHA obtained from the microbial synthesis in the form of an aqueous suspension is preferably subjected to an atomization process by spray drying.

As is known, the spray drying atomization process allows transforming a suspended solid into a liquid medium, usually an aqueous medium, in a dry product in the form of particles with a controlled dimensional distribution. This process generally involves passing the high pressure liquid suspension into a distribution ring, which is provided with a plurality of nozzles from which the suspension comes out in the form of micro-droplets. These micro-droplets are impinged by a jet of hot gas (usually air or nitrogen) which causes almost instantaneous evaporation of the liquid, with the formation of the dried particles that are collected on the bottom of the device. Further details are given, for example, in the manual "Handbook of Industrial Drying", by Arun S. Mujumdar, CRC Press, 4th Edition (2014) .

In this way, it is possible to obtain the PHA in the form of particles having a substantially spherical shape and with an average diameter (d50) of from 0.1 um to 100 um, having a low polydispersion. The polydispersion of the particle size distribution can be calculated as "span", i.e. the ratio (d90 - d10)/d50, where d90 is the diameter value below which 90% by weight of the particle population is found, d10 is the diameter value below which 10% by weight of the particle population is found, and d50 is the diameter value below which 50% by weight of the particle population is found (median value). This ratio (span) preferably has a value of from 1.0 to 2.5.

The PHA particles are characterized by high porosity, with a value of surface area (BET) preferably of from 0.5 to 20 m²/g, more preferably from 1 to 10 m²/g, measured according to the ISO 9277:1995 standard.

The particles of PHA are further characterized by a value of oil absorption (linseed oil) preferably of from 30 to 300 g of oil/100 g of polymer, more preferably from 70 to 130 g of oil/100 g of polymer, measured according to the ISO 787-5:1980 standard.

Preferably, the PHA particles have a structure in the form of hollow capsules, characterized by a substantially compact outer layer and a high-porosity or hollow core.

Preferably, the cosmetic composition comprises:
from 80% by weight to 98% by weight of a cosmetically acceptable base formulation comprising an oily phase;
from 2% by weight to 20% by weight of at least one polyhydroxyalkanoate (PHA) in the form of particles having an average diameter (d50) from 0.5 µm to 100 µm;
the percentages being expressed with respect to the total weight of the composition.

As for the PHA, this is preferably a polymer containing repeating units of formula:

-O-CHR₁-(CH₂)ₙ-CO- (I)

where:
R₁ is selected from -H, C₁-C₁₂ alkyls, C₄-C₁₆ cycloalkyls, C₂-C₁₂ alkenyls, optionally substituted with at least one group selected from: halogen (F, Cl, Br), -CN, -OH, -COOH, -OR, -COOR (R = C₁-C₄ alkyl, benzyl);
n is zero or an integer of from 1 to 6, preferably is 1 or 2.

Preferably, R₁ is methyl or ethyl, and n is 1 or 2.

PHAs can be either homopolymers or copolymers or terpolymers. In the case of copolymers or terpolymers, they may consist of different repeating units of formula (I), or of at least one repeating unit of formula (I) in combination with at least one repeating unit deriving from comonomers that are able to copolymerize with hydroxyalkanoates, such as lactones or lactams. In the latter case, the repeating units of formula (I) are present in an amount equal to at least 10% by moles with respect to the total moles of repeating units.

Particularly preferred repeating units of formula (I) are those deriving from: 3-hydroxybutyrate, 3-hydroxyvalerate, 3-hydroxyhexanoate, 3-hydroxyoctanoate, 3-hydroxyundec-10-enoate, 4-hydroxyvalerate.

Particularly preferred PHAs are: poly-3-hydroxybutyrate (PHB), poly-3-hydroxyvalerate (PHV), poly-3-hydroxyhexanoate (PHH), poly-3-hydroxyoctanoate (PHO), poly(3-hydroxybutyrate-co-3-hydroxyvalerate) (PHBV), poly(3-hydroxybutyrate-co-3-hydroxyexanoate) (PHBH), poly(3-hydroxybutyrate-co-4-hydroxybutyrate), poly(3-hydroxyoctanoate-co-3-hydroxyundecen-10-enoate) (PHOU), poly(3-hydroxybutyrate-co-3-hydroxyvalerate-co-4-hydroxyvalerate) (PHBVV), or mixtures thereof.

Preferably, the PHA has a weight average molecular weight (M_{w}) which can range from 10,000 to 1,000,000 Da.

As for the production of PHA, this is preferably achieved by microbial fermentation of an organic substrate (for example carbohydrates or other fermentable substrates, such as glycerol) through a strain of microorganisms capable of producing PHA, and subsequent recovery of PHA from the cell mass. For further details see, for example patent applications WO 99/23146, WO 2011/045625 and WO 2015/015315. Substrates suitable for the production of PHA by fermentation can be obtained in particular from the processing of vegetables, for example juices, molasses, pulps derived from the processing of sugar beet, sugar cane. These substrates generally contain, in addition to sucrose and other carbohydrates, organic growth factors, nitrogen, phosphorus and/or other minerals useful as nutrients for cell growth. An alternative is glycerol, a low-cost organic carbon source, being a by-product of biodiesel production, which can optionally be used in a mixture with levulinic acid (see, for example, Patent US 8 956 835 B2).

In the context of the present description and of the appended claims, by "average particle diameter" it is meant, unless otherwise indicated, the diameter d50 (median value), that is, the value of the diameter below which 50% by weight of the particle population is found (see "A Guidebook to Particle Size Analysis" published by Horiba Instruments Inc. - 2016, available on https://www.horiba.com/fileadmin/uploads/Scientific/ eMag/PSA/Guidebook/pdf/PSA_Guidebook.pdf). It can be determined by laser diffraction technique, according to the ISO 13320:2009 standard.

With regard to the cosmetically acceptable base formulation, it comprises an oily phase, which is preferably present in an amount of 50% by weight to 100% by weight, more preferably from 70% by weight to 90% by weight, with respect to the weight of the base formulation.

The oily phase generally comprises at least one fatty substance of vegetable, animal, mineral or synthetic origin, which can be in liquid (oils) or solid (waxes) form at room temperature (25 °C). Preferably, the oily phase comprises at least one non-volatile oil which provides an emollient effect on the skin. "Non-volatile oil" means a liquid fatty substance at room temperature (25 °C) which is capable of remaining on the skin after the application for at least one hour, having in particular a vapor pressure at 25 °C and atmospheric pressure less than or equal to 0.01 mmHg (1.33 Pa).

Generally, the oily phase consists of hydrocarbon, silicone or fluorinated compounds, in particular fatty acids, fatty alcohols, fatty acid esters, such as for example: cetearyl isononate, isotridecyl isononate, isostearyl isostearate, isopropyl isostearate, isopropyl myristate, isopropyl palmitate, butyl stearate, hexyl laurate, isononyl isononate, 2-ethylhexyl palmitate, 2-hexhyldecyl laurate, 2-octyldecyl palmitate, 2-octyldodecylic myristate or lactate, of di(2-ethyl-hexyl)succinate, diisostearyl maleate, glyceryl or triglyceryl triisostearate, tocopherol acetate, myristic acid, palmitic acid, stearic acid, behenic acid, oleic acid, linoleic acid, linolenic acid, isostearic acid, dimethicone, triglycerides of caprylic/capric acid, oleic alcohol, avocado oil, camellia oil, castor oil, macadamia nut oil, tortoise oil, mink oil, soybean oil, grapeseed oil, sesame oil, corn oil, canola oil, sunflower seed oil, cottonseed oil, jojoba oil, peanut oil, olive oil, and mixtures thereof.

Mineral oils can also be used, although less preferable from the point of view of environmental sustainability, such as liquid paraffins, squalene, petroleum gels, and mixtures thereof.

The oily phase may also comprise at least one volatile oil, i.e. a liquid fatty substance at room temperature (25°C) which evaporates after the application to the skin in less than one hour, having in particular a vapor pressure at 25°C and atmospheric pressure greater than 0.01 mmHg (1.33 Pa) and generally less than or equal to 300 mmHg (40,000 Pa). The volatile oils can be, for example, silicone oils, which contribute to reducing the oily effect of the fatty phase.

The cosmetically acceptable base formulation may possibly additionally comprise an aqueous phase, which is preferably present in an amount not higher than 50% by weight, more preferably from 10% by weight to 30% by weight, with respect to the weight of the base formulation. The aqueous phase, in addition to water, can comprise products miscible in water, such as: primary and secondary alcohols (e.g. ethanol or isopropanol); polyols (e.g. glycerol, propylene glycol, butylene glycol, dipropylene glycol, diethylene glycol); glycolic ethers (e.g. mono-, di- or tripropylene glycol); mono-, di- or tri-ethylene glycol-C1-C4-alkyl ethers; carboxylic acids (e.g. benzoic acid); or mixtures thereof.

The aqueous phase may also comprise stabilizers, such as sodium chloride, aluminum oxide, magnesium dichloride, magnesium sulfate, zinc dichloride.

The aqueous phase may also comprise other products compatible with the aqueous phase, such as gelling agents, filming agents, thickeners, surfactants, and mixtures thereof.

The cosmetically acceptable base formulation may also comprise other products typically used in cosmetic products, having a purely formulation function which depend on the specific product to be prepared, such as:
surfactants, of the anionic, cationic, amphoteric or non-ionic type, which facilitate the dispersion of the aqueous phase in the oily phase or vice versa, so as to obtain a stable water-in-oil or oil-in-water emulsion;
preservatives, such as parabens and phenoxyethanol;
viscosizing agents, such as gums and waxes, for example hydroxypropylguar, carrageenan, hydroxyethyl cellulose, xanthan gum, guar gum, beeswax, Candelilla wax, ceresin wax, beeswax;
chelating agents, e.g. ethylenediaminetetraacetic acid (EDTA);
dyes and pigments.

The cosmetically acceptable base formulation may also comprise at least one active ingredient having a specific biological activity, such as for example:
moisturizing and nourishing agents, such as amino acids or proteins (glycine, alanine, threonine, citrulline, serine, casein, elastin), alpha-hydroxy acids, sugars (lactose, mannose, fructose, galactose, pectin), vitamins (e.g. vitamin A, D or E);
astringent agents, such as aluminum salts;
antiseptic agents, such as trichlorocarbanilide (TCC), dichlorofen, bromochlorofen, essential oils;
anti-inflammatory and anti-irritant agents, such as alpha-bisabolol, dipotassium glycyrrhizinate, rosemary extracts;
antimicrobial, bacteriostatic, bactericidal, fungistatic or fungicidal agents, such as zinc salts;
anti-cellulite agents, such as caffeine or its derivatives, escin, lecithin, carnitine;
antiseborroic agents, such as iminodibenzyl or fluorine derivatives, glucuronic acid, niacinamides, salicylic acid;
keratolytic or desquamating agents, such as alpha-hydroxyacids, beta-hydroxy acids, alpha-ketoacids, beta-ketoacids, retinoids;
opacifying agents, such as clays, kaolins, antilipase, ethyl lactate;
anti-wrinkle agents, such as proteins and their hydrolysates, collagen, hyaluronic acid;
antioxidants, such as butylated hydroxytoluene (BHT), butylated hydroxyanisole (BHA), propyl gallate, octyl gallate, carotenoids, such as beta-carotene, lycopene or canthaxanthin, ubiquinone, vitamin E, vitamin C;
agents to combat free radicals, such as vitamin E, caffeine, mannitol, enzymes;
sunscreens, such as UV-A and/or UV-B absorbing agents, pigments or nanopigments;
vasoconstrictors;
microcirculation reactivating agents;
decongestants;
deodorants, perfumes and antiperspirants;
depilatory agents, such as thioglycolic and thiolactic acid salts.

The cosmetic composition according to the present invention can be used to produce a wide range of cosmetic products, and in particular:
face make-up products, such as foundations, eyeshadows, mascaras, eyeliners, lipsticks;
face and body care products, such as creams, cosmetic milks, lotions, cleansing products, deodorants, antiperspirants, shaving products, depilatory products;
hair care products, such as shampoo, gels, anti-dandruff products, products against hair loss, products for dyeing or decolouring hair;
perfumery products, particularly in the form of cosmetic milk or cream.

The following embodiment examples are provided purely for illustrative purposes of the present invention

### EXAMPLE 1.

An aqueous dispersion of PHA (concentration of about 20% by weight), obtained from the bacterial fermentation process and previously subjected to purification and bleaching, was subjected to atomization with a high pressure spray dryer, in an open loop configuration. The drying gas was air, with an inlet temperature of 200 °C and flow of 1 m³/min. The PHA powder thus obtained had the following features:
- average diameter (d50) = 10 µm (ISO 13320:2009)
- "span" = (d90 - d10)/d50 = 1.4 (ISO 13320:2009);
- absorption of linseed oil = 106 g of oil/100 g of polymer (ISO 787-5:1980).

### EXAMPLE 2.

The ability to adsorb oily substances by PHA particles prepared according to Example 1 was evaluated.

The tests were carried out by placing a known amount of PHA in powder, deposited on an aluminum sheet, in contact with the oil, which was added dropwise until the PHA powder was able to absorb it. When it was observed that the mixture exuded oil, the test was terminated and the powder mixture of PHA and oil was weighed, so as to determine the amount of oil absorbed by difference.

The results are shown in the following Table 1, where the oil adsorbent capacity is measured as grams of oil adsorbed per 100 grams of PHA.

**TABLE 1**

| OIL | ADSORBENT CAPACITY (g/100 g) |
|---|---|
| caprylic/capric triglyceride | 174.4 |
| dicapril ether | 178.8 |
| paraffin | 90.0 |
| dimethicone | 82.4 |

### EXAMPLE 3.

The compatibility of the powder PHA of Example 1 with some viscosizing agents commonly used in cosmetic formulations was evaluated.

The tests were carried out by adding increasing amounts of PHA in powder (Example 1) to the viscosizing agent dispersed in water. The resulting suspension, maintained at a temperature of 25 °C, was evaluated by a Brookfield RVT viscometer (speed gradient: 5.0 rpm), equipped with Helipath accessory for Brookfield viscosity measurement. The results are shown in Table 2.

**TABLE 2**

| Viscosizing agent | PHA (% by weight) | Brookfield viscosity (mPa.sec) |
|---|---|---|
| hydroxypropylguar (1% by weight) | 0 | 6320 |
| | 1 | 5880 |
| | 5 | 7080 |
| | 10 | 13000 |
| carrageenan (1% by weight) | 0 | 1502 |
| | 1 | 1200 |
| | 5 | 1328 |
| | 10 | 1216 |
| hydroxyethylcellulose (1% by weight) | 0 | 936 |
| | 1 | 800 |
| | 5 | 744 |
| | 10 | 1880 |
| xanthan gum (0.5% by weight) | 0 | 3760 |
| | 1 | 2272 |
| | 5 | 2600 |
| | 10 | 5216 |

As can be seen from the data shown in Table 2, the addition of the powdered PHA did not lead to an excessive increase in the viscosity of the gel, confirming the high compatibility of the PHA itself with the viscosizing agent.

### EXAMPLE 4.

Some lipsticks were prepared with the following base formulation (% by weight with respect to the total weight of the base formulation):

| | |
|---|---|
| *- Ricinus communis* seed oil | 15.30% |
| - diisostearyl maleate | 15.00% |
| - phenyltrimethicone | 5.00% |
| - titanium dioxide (CI 77891) | 4.00% |
| - iron oxides (CI 77491) | 3.00% |
| - red pigment 57 (CI 15850) | 1.00% |
| - octyldodecyl ricinoleate | 15.00% |
| - butylated hydroxytoluene (BHT) | 0.10% |
| - polyethylene | 2.00% |
| - *Limnanthes alba* seed oil | 10.00% |

### Butyrospermum parkii butter extract

| | |
|---|---|
| - beeswax | 6.80% |
| - Candelilla wax | 9.00% |
| - ceresin wax (ozokerite) | 6.80% |
| - C10-C30 esters of cholesterol/lanosterol | 6.50% |
| - tocopheryl acetate | |

The base formulation was compared to the same formulation admixed with 1% by weight and 3% by weight (with respect to the total weight of the composition) of powdered PHA obtained according to Example 1. With the three different formulations lipsticks in stick were produced, which were evaluated for:
- writing characteristics;
- breaking load;
- drop point.

The writing characteristics were empirically evaluated by applying the lipstick on the inside of the forearm; the parameters considered were smoothness (absence of friction), homogeneity of the stroke (uniformity of pigmentation) and opacity (ability to cover the skin coloring). The results are shown in Table 3, compared to the base formulation without PHA.

**TABLE 3**

| Parameter | PHA 1% | PHA 3% |
|---|---|---|
| Smoothness | improved | much improved |
| Homogeneity of the stroke | improved | much improved |
| Opacity | improved | much improved |

The breaking load was evaluated empirically by fixing one end of the stick, to which an increasing weight was applied by the gradual filling of a beaker hanging from the median part of the stick by adhesive tape, until the stick itself breaks. A load of about 200-300 g is generally considered satisfactory, while a load of less than 200 g is an indication of stick fragility. Breaking load values above 300 g are generally indicative of poor pastel softness. The results are shown in Table 4.

**TABLE 4**

| PHA (% weight) | Average breaking load (g) ± standard deviation |
|---|---|
| 0 | 207 ± 19.3 |
| 1 | 132 ± 122.0 (p<0. 05) |
| 3 | 268 ± 27.9 (p=0.15) |

As can be seen from the results shown in Table 4, the addition of PHA in small amounts (1% by weight) reduced the mechanical resistance of the stick, while for higher amounts (3% by weight) a significant strengthening was observed.

The drop point is the temperature at which the stick passes from the semi-solid to the semi-liquid phase and starts to drip (determinable by the AOCS Standard Procedure Cc 18-80 method). Usually it is believed that a drop point between 65°C and 72°C is optimal as it avoids excessive softening in conditions of high ambient temperatures, while higher values may indicate poor applicative properties due to excessive hardness at room temperature. The results obtained are shown in Table 5.

**TABLE 5**

| PHA (% weight) | Drop point (°C) |
|---|---|
| 0 | 60 |
| 1 | 65 |
| 3 | 68 |

### EXAMPLE 5.

Some mascara composition were prepared with the following base formulation (% by weight with respect to the total weight of the base formulation):

| | |
|---|---|
| - water | 54.65% |
| - water, trisodium ethylenediamine Disuccinate | 0.30% |
| - triethanolamine | 1.50% |
| - methoxymethylbutanol | 3.00% |
| - magnesium aluminum silicate | 1.50% |
| - hydroxyethyl cellulose | 0.20% |
| - isostearic acid | 1.00% |
| - butyleneglycol dicaprylate/dicaprate | 3.00% |
| - stearic acid | 3.00% |
| - glyceryl stearate SE | 1.20% |
| - PVP/eicosene copolymer | 5.00% |
| - microcrystalline wax | 4.20% |
| - Candelilla wax | 4.20% |
| - synthetic beeswax | 4.20% |
| - butylated hydroxytoluene (BHT) | 0.05% |
| - methyl paraben | 0.15% |
| - ethylparaben | 0.15% |
| - polysorbate-20 | 0.50% |
| - black ferrous/ferric oxide (CI 77499) | 10.00% |
| - phenoxyethanol | 0.60% |
| - o-cimen-5-ol | 0.10% |
| - mica | 0.50% |

The base formulation was compared to the same formulation admixed with 2% by weight and 4% by weight (with respect to the total weight of the composition) of powdered PHA obtained according to Example 1. The three different mascara formulations were evaluated by Brookfield viscosity measurements at 25°C as described in Example 3, using two different velocity gradients (2.5 rpm and 5.0 rpm). The results are shown in Table 6.

**TABLE 6**

| PHA (% weight) | Brookfield viscosity @ 2.5 rpm (mPa.sec) | Brookfield viscosity @ 5.0 rpm (mPa.sec) |
|---|---|---|
| 0 | 290,000 | 150,000 |
| 1 | 220,000 | 120,000 |
| 3 | 290,000 | 150,000 |

As can be seen from the results shown in Table 6, small amounts of PHA induced a slight decrease in viscosity, which increased with higher percentages.

The same mascara compositions were also evaluated empirically and the results of the compositions containing PHA are shown in Table 7, compared with the PHA-free composition.

**TABLE 7**

| Parameter | PHA 2% | PHA 4% |
|---|---|---|
| Macroscopic appearance | more shiny and fluid | more shiny |
| Voluminizing effect | unchanged | unchanged |
| Lenghtening effect | a little higher | higher |
| Drying speed | unchanged | unchanged |
| Curling effect | unchanged | higher |

As can be seen from the results shown in Table 7, the introduction of PHA in the mascara composition significantly improves the shine, the lengthening effect and the curling effect thereof, especially with a higher amount of PHA (4% by weight).

## Claims

1. Cosmetic composition comprising:
from 70% by weight to 99.9% by weight of a cosmetically acceptable base formulation comprising an oily phase;
from 0.1% by weight to 30% by weight of at least one polyhydroxyalkanoate (PHA) in the form of particles having an average diameter (d50) from 0.1 µm to 100 µm;
the percentages being expressed with respect to the total weight of the composition;
wherein the particles of PHA are obtained by a process of atomization by spray drying of an aqueous suspension of PHA.

2. Cosmetic composition according to claim 1, wherein the particles of PHA have a substantially spherical form.

3. Cosmetic composition according to any one of the preceding claims, wherein the particles of PHA have an average diameter (d50) from 1 µm to 50 µm, preferably from 5 µm to 30 µm.

4. Cosmetic composition according to any one of the preceding claims, wherein the particles of PHA have a value of (d90 - d10)/d50 ratio (span) from 1.0 to 2.5.

5. Cosmetic composition according to any one of the preceding claims, wherein the particles of PHA have a value of surface area (BET) from 0.5 to 20 m²/g, preferably from 1 to 10 m²/g, measured according to standard ISO 9277:1995.

6. Cosmetic composition according to any one of the preceding claims, wherein the particles of PHA have a value of oil absorption (linseed oil) from 30 to 300 g of oil/100 g of polymer, preferably form 70 to 130 g of oil/100 g of polymer, measured according to standard ISO 787-5:1980.

7. Cosmetic composition according to any one of the preceding claims, wherein the particles of PHA have a structure in the form of hollow capsules.

8. Cosmetic composition according to any one of the preceding claims, comprising:
from 80% by weight to 98% by weight of a cosmetically acceptable base formulation comprising an oily phase;
from 2% by weight to 20% by weight of at least one polyhydroxyalkanoate (PHA) in the form of particles having an average diameter from 0.5 µm to 100 µm;
the percentages being expressed with respect to the total weight of the composition.

9. Cosmetic composition according to any one of the preceding claims, wherein the PHA is selected from: poly-3-hydroxybutyrate (PHB), poly-3-hydroxyvalerate (PHV), poly-3-hydroxyhexanoate (PHH), poly-3-hydroxyoctanoate (PHO), poly(3-hydroxybutyrate-co-3-hydroxyvalerate) (PHBV), poly(3-hydroxybutyrate-co-3-hydroxyexanoate) (PHBH), poly(3-hydroxybutyrate-co-4-hydroxybutyrate), poly(3-hydroxyoctanoate-co-3-hydroxyundecen-10-enoate) (PHOU), poly(3-hydroxybutyrate-co-3-hydroxyvalerate-co-4-hydroxyvalerate) (PHBVV), or mixtures thereof.

10. Cosmetic composition according to any one of the preceding claims, wherein the PHA has a weight average molecular weight (M_{w}) from 10,000 to 1,000,000 Da.

11. Cosmetic composition according to any one of the preceding claims, wherein the cosmetically acceptable base formulation comprises an oily phase in an amount from 50% by weight to 100% by weight, preferably from 70% by weight to 90% by weight, with respect to the weight of the base formulation.

12. Cosmetic composition according to any one of the preceding claims, wherein the oily phase comprises at least one non-volatile oil, liquid at room temperature (25°C), having a vapor pressure at 25°C and atmospheric pressure lower than or equal to 0.01 mmHg (1.33 Pa).

13. Cosmetic composition according to any one of the preceding claims, wherein the oily phase comprises at least one volatile oil, liquid at room temperature (25°C), having a vapor pressure at 25°C and atmospheric pressure higher than 0.01 mmHg (1.33 Pa) and lower than or equal to 300 mmHg (40,000 Pa).

14. Cosmetic composition according to any one of the preceding claims, wherein the cosmetically acceptable base formulation further comprises an aqueous phase.

15. Cosmetic composition according to claim 14, wherein the aqueous phase is present in an amount not higher than 50% by weight, preferably from 10% by weight to 30% by weight, with respect to weight of the base formulation.

16. Use of a cosmetic composition according to any one of the claims from 1 to 15 for producing a cosmetic product selected from:
face make-up products, such as foundations, eyeshadows, mascaras, eyeliners, lipsticks;
face and body care products, such as creams, cosmetic milks, lotions, cleansing products, deodorants, antiperspirants, shaving products, depilatory products;
hair care products, such as shampoo, gels, anti-dandruff products, products against hair loss, products for dyeing or decolouring hair;
perfumery products, particularly in the form of cosmetic milk or cream.

## Patentansprüche

1. Kosmetische Zusammensetzung, umfassend:
von 70 Gew.- % bis 99,9 Gew.- % einer kosmetisch akzeptablen Basisformulierung, die eine Ölphase umfasst;
von 0,1 Gew.- % bis 30 Gew.- % mindestens eines Polyhydroxyalkanoats (PHA) in Form von Partikeln mit einem durchschnittlichen Durchmesser (d50) von 0,1 µm bis 100 µm;
wobei die Prozentsätze in Bezug auf das Gesamtgewicht der Zusammensetzung angegeben werden;
wobei die PHA-Partikel durch ein Verfahren zur Zerstäubung durch Sprühtrocknung einer wässrigen PHA-Suspension erhalten werden.

2. Kosmetische Zusammensetzung nach Anspruch 1, wobei die PHA-Partikel eine im Wesentlichen kugelförmige Form aufweisen.

3. Kosmetische Zusammensetzung nach einem der vorhergehenden Ansprüche, wobei die PHA-Partikel einen durchschnittlichen Durchmesser (d50) von 1 µm bis 50 µm, vorzugsweise von 5 µm bis 30 µm aufweisen.

4. Kosmetische Zusammensetzung nach einem der vorhergehenden Ansprüche, wobei die PHA-Partikel einen Wert von (d90 - d10)/d50-Verhältnis (Spanne) von 1,0 bis 2,5 aufweisen.

5. Kosmetische Zusammensetzung nach einem der vorhergehenden Ansprüche, wobei die PHA-Partikel einen Oberflächenwert (BET) von 0,5 bis 20 m²/g, vorzugsweise von 1 bis 10 m²/g, gemessen gemäß der Norm ISO 9277:1995, aufweisen.

6. Kosmetische Zusammensetzung nach einem der vorhergehenden Ansprüche, wobei die PHA-Partikel einen Wert der Ölabsorption (Leinöl) von 30 bis 300 g von ÖI/100 g von Polymer, vorzugsweise von 70 bis 130 g von ÖI/100 g Polymer, gemessen gemäß der Norm ISO 787-5:1980, aufweisen.

7. Kosmetische Zusammensetzung nach einem der vorhergehenden Ansprüche, wobei die PHA-Partikel eine Struktur in Form von Hohlkapseln aufweisen.

8. Kosmetische Zusammensetzung nach einem der vorhergehenden Ansprüche, umfassend:
von 80 Gew.- % bis 98 Gew.- % einer kosmetisch akzeptablen Basisformulierung, die eine Ölphase umfasst;
von 2 Gew.- % bis 20 Gew.- % mindestens eines Polyhydroxyalkanoats (PHA) in Form von Partikeln mit einem durchschnittlichen Durchmesser von 0,5 µm bis 100 µm;
wobei die Prozentsätze in Bezug auf das Gesamtgewicht der Zusammensetzung angegeben werden.

9. Kosmetische Zusammensetzung nach einem der vorhergehenden Ansprüche, wobei das PHA ausgewählt ist aus: Poly-3-hydroxybutyrat (PHB), Poly-3-hydroxyvalerat (PHV), Poly-3-hydroxyhexanoat (PHH), Poly-3-hydroxyoctanoat (PHO), Poly(3-hydroxybutyrat-co-3-hydroxyvalerat) (PHBV), Poly(3-hydroxybutyrat-co-3-hydroxyhexanoat) (PHBH), Poly(3-hydroxybutyrat-co-4-hydroxybutyrat), Poly(3-hydroxyoctanoat-co-3-hydroxyundecen-10-enoat) (PHOU), Poly(3-hydroxybutyrat-co-3-hydroxyvalerat-co-4-hydroxyvalerat) (PHBVV) oder Gemischen davon.

10. Kosmetische Zusammensetzung nach einem der vorhergehenden Ansprüche, wobei das PHA ein Gewichtsmittel des Molekulargewichts (Mw) von 10.000 bis 1.000.000 Da aufweist.

11. Kosmetische Zusammensetzung nach einem der vorhergehenden Ansprüche, wobei die kosmetisch akzeptable Basisformulierung eine Ölphase in einer Menge von 50 Gew.- % bis 100 Gew.- %, vorzugsweise von 70 Gew.- % bis 90 Gew.- %, bezogen auf das Gewicht der Basisformulierung, umfasst.

12. Kosmetische Zusammensetzung nach einem der vorhergehenden Ansprüche, wobei die Ölphase mindestens ein nichtflüchtiges Öl umfasst, das bei Raumtemperatur (25°C) flüssig ist und einen Dampfdruck bei 25 °C und einen Atmosphärendruck von weniger als oder gleich 0,01 mmHg (1,33 Pa) aufweist.

13. Kosmetische Zusammensetzung nach einem der vorhergehenden Ansprüche, wobei die Ölphase mindestens ein flüchtiges Öl umfasst, das bei Raumtemperatur (25 °C) flüssig ist und einen Dampfdruck bei 25 °C und Atmosphärendruck von mehr als 0,01 mmHg (1,33 Pa) und weniger als oder gleich 300 mmHg (40.000 Pa) aufweist.

14. Kosmetische Zusammensetzung nach einem der vorhergehenden Ansprüche, wobei die kosmetisch akzeptable Basisformulierung ferner eine wässrige Phase umfasst.

15. Kosmetische Zusammensetzung nach Anspruch 14, wobei die wässrige Phase in einer Menge von nicht mehr als 50 Gew.- %, vorzugsweise von 10 Gew.- % bis 30 Gew.- %, bezogen auf das Gewicht der Basisformulierung, vorhanden ist.

16. Verwendung einer kosmetischen Zusammensetzung nach einem der Ansprüche 1 bis 15 zur Herstellung eines kosmetischen Produkts, ausgewählt aus:
Make-up-Produkten für das Gesicht, wie Grundierungen, Lidschatten, Mascaras, Eyeliner, Lippenstiften;
Gesichts- und Körperpflegeprodukten, wie Cremes, kosmetische Milch, Lotionen, Reinigungsprodukte, Deodorants, Antitranspirantien, Rasierprodukte, Enthaarungsprodukte;
Haarpflegeprodukten wie Shampoo, Gele, Antischuppenprodukte, Produkte gegen Haarausfall, Produkte zum Färben oder Entfärben von Haar;
Parfümerieprodukten, insbesondere in Form von kosmetischer Milch oder Creme.

## Revendications

1. Composition cosmétique comprenant:
de 70% en poids à 99,9% en poids d'une formulation de base cosmétiquement acceptable comprenant une phase huileuse;
de 0,1% en poids à 30% en poids d'au moins un polyhydroxyalcanoate (PHA) sous forme de particules ayant un diamètre moyen (d50) de 0,1 µm à 100 µm;
les pourcentages étant exprimés par rapport au poids total de la composition;
dans lequel les particules de PHA sont obtenues par un processus d'atomisation par séchage par atomisation d'une suspension aqueuse de PHA.

2. Composition cosmétique selon la revendication 1, dans laquelle les particules de PHA ont une forme sensiblement sphérique.

3. Composition cosmétique selon l'une quelconque des revendications précédentes, dans laquelle les particules de PHA ont un diamètre moyen (d50) de 1 µm à 50 µm, de préférence de 5 µm à 30 µm.

4. Composition cosmétique selon l'une quelconque des revendications précédentes, dans laquelle les particules de PHA ont une valeur du rapport (d90 - d10)/d50 (span) de 1,0 à 2,5.

5. Composition cosmétique selon l'une quelconque des revendications précédentes, dans laquelle les particules de PHA ont une valeur de surface (BET) de 0,5 à 20 m²/g, de préférence de 1 à 10 m²/g, mesurée selon la norme ISO 9277:1995.

6. Composition cosmétique selon l'une quelconque des revendications précédentes, dans laquelle les particules de PHA ont une valeur d'absorption d'huile (huile de lin) de 30 à 300 g d'huile/100 g de polymère, de préférence de 70 à 130 g d'huile/100 g de polymère, mesurée selon la norme ISO 787-5:1980.

7. Composition cosmétique selon l'une quelconque des revendications précédentes, dans laquelle les particules de PHA ont une structure en forme de capsules creuses.

8. Composition cosmétique selon l'une quelconque des revendications précédentes, comprenant:
de 80% en poids à 98% en poids d'une formulation de base cosmétiquement acceptable comprenant une phase huileuse;
de 2% en poids à 20% en poids d'au moins un polyhydroxyalcanoate (PHA) sous forme de particules ayant un diamètre moyen de 0,5 µm à 100 µm;
les pourcentages étant exprimés par rapport au poids total de la composition.

9. Composition cosmétique selon l'une quelconque des revendications précédentes, dans laquelle le PHA est choisi parmi: poly-3-hydroxybutyrate (PHB), poly-3-hydroxyvalérate (PHV), poly-3-hydroxyhexanoate (PHH), poly-3-hydroxyoctanoate (PHO), poly(3-hydroxybutyrate-co-3-hydroxyvalérate) (PHBV), poly(3-hydroxybutyrate-co-3-hydroxyexanoate) (PHBH), poly(3-hydroxybutyrate-co-4-hydroxybutyrate), poly(3-hydroxyoctanoate-co-3-hydroxyundecen-10-enoate) (PHOU), poly(3-hydroxybutyrate-co-3-hydroxyvalérate-co-4-hydroxyvalérate) (PHBVV), ou leurs mélanges.

10. Composition cosmétique selon l'une quelconque des revendications précédentes, dans laquelle le PHA a un poids moléculaire moyen en poids (Mw) de 10 000 à 1 000 000 Da.

11. Composition cosmétique selon l'une quelconque des revendications précédentes, dans laquelle la formulation de base cosmétiquement acceptable comprend une phase huileuse en une quantité allant de 50% en poids à 100% en poids, de préférence de 70% en poids à 90% en poids, par rapport au poids de la formulation de base.

12. Composition cosmétique selon l'une quelconque des revendications précédentes, dans laquelle la phase huileuse comprend au moins une huile non volatile, liquide à température ambiante (25°C), ayant une pression de vapeur à 25°C et à la pression atmosphérique inférieure ou égale à 0,01 mmHg (1,33 Pa).

13. Composition cosmétique selon l'une quelconque des revendications précédentes, dans laquelle la phase huileuse comprend au moins une huile volatile, liquide à température ambiante (25°C), ayant une pression de vapeur à 25°C et à la pression atmosphérique supérieure à 0,01 mmHg (1,33 Pa) et inférieure ou égale à 300 mmHg (40 000 Pa).

14. Composition cosmétique selon l'une quelconque des revendications précédentes, dans laquelle la formulation de base cosmétiquement acceptable comprend en outre une phase aqueuse.

15. Composition cosmétique selon la revendication 14, dans laquelle la phase aqueuse est présente en une quantité ne dépassant pas 50% en poids, de préférence de 10% en poids à 30% en poids, par rapport au poids de la formulation de base.

16. Utilisation d'une composition cosmétique selon l'une quelconque des revendications de 1 à 15 pour produire un produit cosmétique choisi parmi:
les produits de maquillage pour le visage, tels que les fonds de teint, les fards à paupières, les mascaras, les eye-liners et les rouges à lèvres;
les produits de soins du visage et du corps, tels que les crèmes, les laits cosmétiques, les lotions, les produits nettoyants, les déodorants, les antisudorifiques, les produits de rasage, les produits dépilatoires;
les produits de soins capillaires, tels que les shampooings, les gels, les produits antipelliculaires, les produits contre la chute des cheveux, les produits pour la teinture ou la décoloration des cheveux;
les produits de parfumerie, notamment sous forme de lait ou de crème cosmétique.
